# EUROPEAN PATENT APPLICATION

(11) **EP 4 270 400 A1**
(43) Date of publication of application: **01.11.2023**
(21) Application number: 21910122.7
(22) Date of filing: 25.11.2021
(51) Int. Cl.: G16C 20/50, G16C 20/40

(54) **INFORMATION PROCESSING DEVICE, INFORMATION PROCESSING METHOD, AND INFORMATION PROCESSING PROGRAM**

(30) Priority: 25.12.2020 JP 2020216937
(71) Applicant: FUJIFILM Corporation, Tokyo 106-8620 (JP)
(72) Inventor: YARIMIZU, Hirokazu, Tokyo 106-8620 (JP); HIKIDA, Yasushi, Tokyo 106-8620 (JP)
(74) Representative: Meissner Bolte Partnerschaft mbB
(86) International application number: PCT/JP2021/043215
(87) International publication number: WO 2022/137969

(57) **Abstract**

An information processing apparatus performs control of displaying a structure of a chemical substance, which is a processing target, and a first index value indicating performance or the structure of the chemical substance on a display device, receives a change operation with respect to the structure of the chemical substance displayed on the display device, and performs control of displaying a structure of the chemical substance after the change operation and a second index value indicating performance or the structure of the chemical substance after the change operation on the display device, and performs, in a case in which a partial structure that contributes to a change in a value from the first index value to the second index value is present in the chemical substance after the change operation, control of highlighting the partial structure.

## Description

### Technical Field

The present disclosure relates to an information processing apparatus, an information processing method, and an information processing program.

### Related Art

JP1989-161578A (JP-H01-161578A) discloses that in order to support molecular design, a first chemical structural formula is displayed, a second chemical structural formula obtained by complementing a partial structure substituted in the displayed first chemical structural formula is displayed, and the characteristics of the second chemical structural formula are displayed.

### SUMMARY OF INVENTION

### Technical Problem

At a stage of design of a chemical substance, a designer uses a molecular design editor to design the structure of the chemical substance. Examples of the molecular design editor include ChemDraw (registered trademark).

However, in the existing molecular design editor, since a main function is a molecular design function, in a case in which the structure of the chemical substance is changed on the molecular design editor, it is difficult to understand how the change influences the index value of the chemical substance.

### Solution to Problem

The present disclosure has been made in view of the above circumstances, and the present disclosure is to provide an information processing apparatus, an information processing method, and an information processing program capable of understanding an influence of a chemical substance on an index value in a case in which a structure of the chemical substance is changed.

The present disclosure relates to an information processing apparatus comprising at least one processor, in which the processor performs control of displaying a structure of a chemical substance, which is a processing target, and a first index value indicating performance or the structure of the chemical substance on a display device, receives a change operation with respect to the structure of the chemical substance displayed on the display device, and performs control of displaying a structure of the chemical substance after the change operation and a second index value indicating performance or the structure of the chemical substance after the change operation on the display device, and performs, in a case in which a partial structure that contributes to a change in a value from the first index value to the second index value is present in the chemical substance after the change operation, control of highlighting the partial structure.

It should be noted that, in the information processing apparatus according to the present disclosure, the processor may perform control of displaying both the first index value and the second index value in a case in which the first index value and the second index value are different from each other.

In addition, in the information processing apparatus according to the present disclosure, the processor may determine that the partial structure that contributes to the change in the value from the first index value to the second index value is present, in a case in which the partial structure included in the chemical substance after the change operation is present in partial structure data in which a partial structure of the chemical substance and an index value indicating performance or a structure of the partial structure are associated with each other.

In addition, in the information processing apparatus according to the present disclosure, the processor may further perform control of highlighting a portion in which the structure of the chemical substance is changed before and after the change operation.

In addition, the present disclosure relates to an information processing method executed by a processor provided in an information processing apparatus, the method comprising performing control of displaying a structure of a chemical substance, which is a processing target, and a first index value indicating performance or the structure of the chemical substance on a display device, receiving a change operation with respect to the structure of the chemical substance displayed on the display device, and performing control of displaying a structure of the chemical substance after the change operation and a second index value indicating performance or the structure of the chemical substance after the change operation on the display device, and performing, in a case in which a partial structure that contributes to a change in a value from the first index value to the second index value is present in the chemical substance after the change operation, control of highlighting the partial structure.

In addition, the present disclosure relates to an information processing program causing a processor provided in an information processing apparatus to execute a process comprising performing control of displaying a structure of a chemical substance, which is a processing target, and a first index value indicating performance or the structure of the chemical substance on a display device, receiving a change operation with respect to the structure of the chemical substance displayed on the display device, and performing control of displaying a structure of the chemical substance after the change operation and a second index value indicating performance or the structure of the chemical substance after the change operation on the display device, and performing, in a case in which a partial structure that contributes to a change in a value from the first index value to the second index value is present in the chemical substance after the change operation, control of highlighting the partial structure.

### Effects of Invention

According to the present disclosure, it is possible to understand the influence of the chemical substance on the index value in a case in which the structure of the chemical substance is changed.

### BRIEF DESCRIPTION OF THE DRAWINGS

Fig. 1 is a block diagram showing an example of a hardware configuration of an information processing apparatus.
Fig. 2 is a diagram showing a graph showing a structure of a chemical substance.
Fig. 3 is a diagram for describing partial structure data.
Fig. 4 is a diagram showing an example of the partial structure data.
Fig. 5 is a block diagram showing an example of a functional configuration of the information processing apparatus.
Fig. 6 is a diagram showing an example of a first display screen.
Fig. 7 is a diagram showing an example of a second display screen.
Fig. 8 is a flowchart showing an example of highlighting processing.
Fig. 9 is a diagram for describing a specific example.
Fig. 10 is a diagram for describing the specific example.

### DESCRIPTION OF EMBODIMENTS

Hereinafter, with reference to the drawings, an embodiment for performing the technology of the present disclosure will be described in detail.

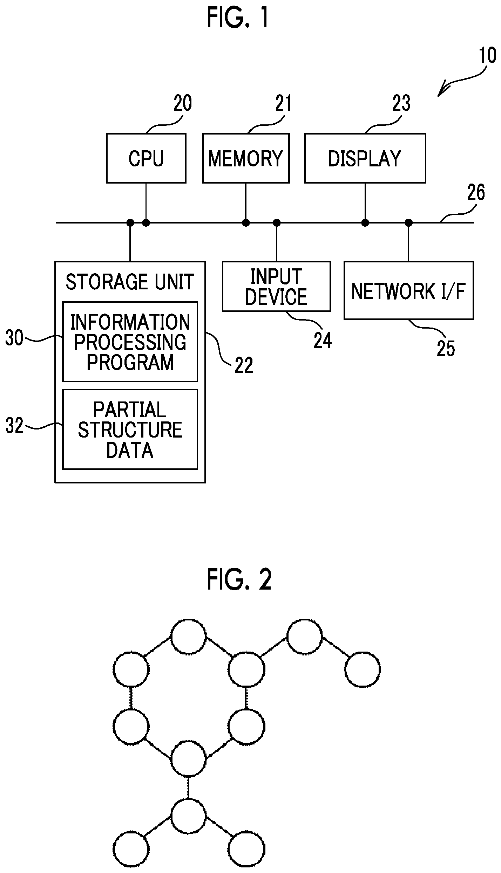

First, with reference to Fig. 1, a hardware configuration of an information processing apparatus 10 according to the present embodiment will be described. As shown in Fig. 1, the information processing apparatus 10 includes a central processing unit (CPU) 20, a memory 21 as a transitory storage area, and a non-volatile storage unit 22. Moreover, the information processing apparatus 10 includes a display 23, such as a liquid crystal display, an input device 24, such as a keyboard and a mouse, and a network interface (I/F) 25 connected to a network. The CPU 20, the memory 21, the storage unit 22, the display 23, the input device 24, and the network I/F 25 are connected to a bus 26. Examples of the information processing apparatus 10 include a personal computer and a server computer.

The storage unit 22 is realized by a hard disk drive (HDD), a solid state drive (SSD), a flash memory, or the like. An information processing program 30 is stored in the storage unit 22 as a storage medium. The CPU 20 reads out the information processing program 30 from the storage unit 22, develops the read out information processing program 30 in the memory 21, and executes the developed information processing program 30.

In addition, partial structure data 32 is stored in the storage unit 22. With reference to Figs. 2 to 4, the partial structure data 32 will be described.

As shown in Fig. 2 as an example, the information processing apparatus 10 according to the present embodiment handles structure data in which a structure of a chemical substance is represented in a graph format as structure data indicating the structure of the chemical substance. In the structure data, atoms are represented as nodes and bonds are represented as edges. It should be noted that the format of the structure data is not limited to the graph format. For example, as the format of the structure data, a character string format such as a deoxyribonucleic acid (DNA) base sequence may be applied.

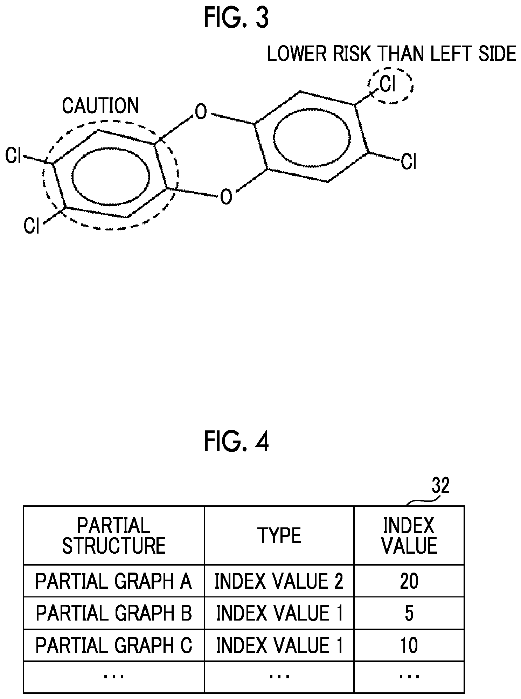

In addition, as shown in Fig. 3 as an example, a part of the structure constituting the chemical substance may influence an index value indicating the performance or the structure of the chemical substance. In the following description, a part of the structure constituting the chemical substance will be referred to as a "partial structure". Examples of the index value indicating the performance of the chemical substance include the presence or absence of carcinogenicity, the presence or absence of toxicity, and a degree of solubility in water. In addition, examples of the index value indicating the structure of the chemical substance include a molecular weight and the number of ring structures. In the following description, in a case in which the index value indicating the performance of the chemical substance and the index value indicating the structure are generically referred to, both index values are simply referred to as an "index value".

Fig. 4 shows an example of the partial structure data 32. As shown in Fig. 4, in the partial structure data 32, the partial structure of the chemical substance is associated with a type of the index value of the partial structure and the index value. In addition, the partial structure data 32 includes a plurality of combinations of the partial structure, and the type of the index value of the partial structure and the index value. A partial graph in Fig. 4 means the structure data in which the partial structure is represented in a graph format. For example, the partial structure data 32 is created in advance based on the experience and knowledge of an expert. It should be noted that, in the partial structure data 32, one partial structure may be associated with one type of index value, or may be associated with a plurality of types of index values.

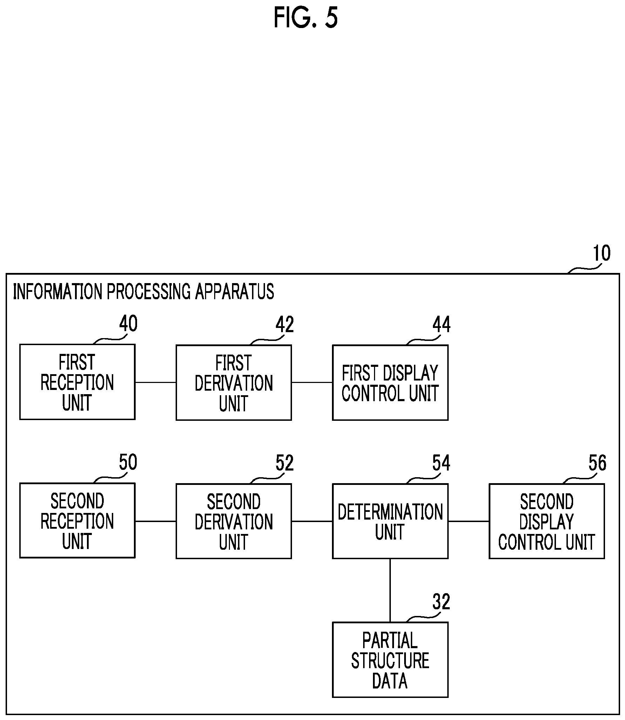

Next, with reference to Fig. 5, a functional configuration of the information processing apparatus 10 according to the present embodiment will be described. As shown in Fig. 5, the information processing apparatus 10 includes a first reception unit 40, a first derivation unit 42, a first display control unit 44, a second reception unit 50, a second derivation unit 52, a determination unit 54, and a second display control unit 56. The CPU 20 executes the information processing program 30 to function as the first reception unit 40, the first derivation unit 42, the first display control unit 44, the second reception unit 50, the second derivation unit 52, the determination unit 54, and the second display control unit 56.

The first reception unit 40 receives the structure data indicating the structure of the chemical substance, which is a processing target and is designed by a user using a molecular design editor. The first derivation unit 42 derives a first index value indicating the performance or the structure of the chemical substance, which is the processing target, based on the structure data received by the first reception unit 40. For example, the first derivation unit 42 derives the first index value based on an actual measurement value obtained in the past experiment. It should be noted that, in a case in which the actual measurement value is not present, the first derivation unit 42 may derive the first index value by, for example, a known method such as a quantitative structure-activity relationship (QSAR).

The first display control unit 44 performs control of displaying the structure of the chemical substance, which is the processing target, indicated by the structure data received by the first reception unit 40 and the first index value of the chemical substance derived by the first derivation unit 42 on the display 23.

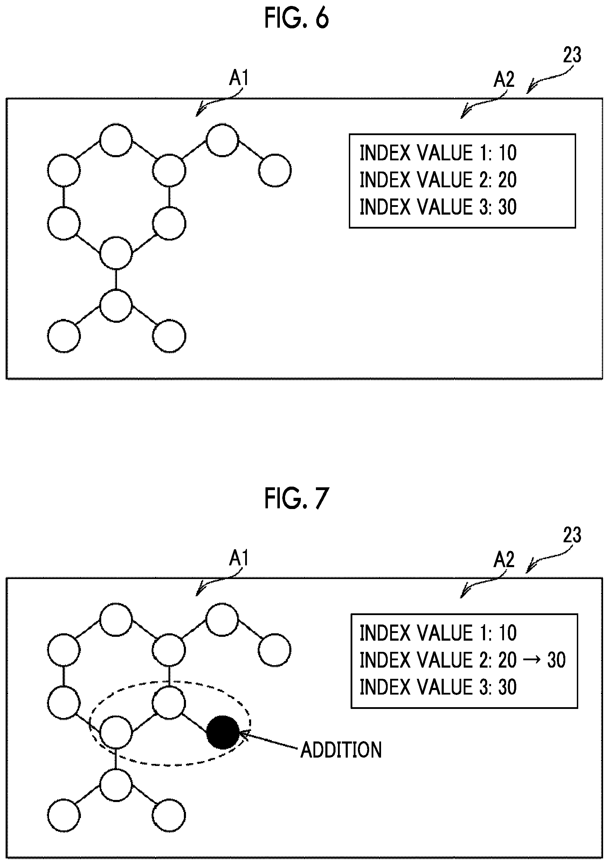

Fig. 6 shows an example of a first display screen displayed on the display 23 by the control of the first display control unit 44. As shown in Fig. 6, the first display screen according to the present embodiment includes two display regions A1 and A2. A graph showing the structure of the chemical substance, which is the processing target, is displayed in the display region A1. The first index value of the chemical substance, which is the processing target, is displayed in the display region A2. In Fig. 6, an example is shown in which three types of first index values are displayed.

The user performs a change operation with respect to the structure of the chemical substance displayed on the display 23. Examples of the change operation include addition, substitution, and deletion of nodes and edges. The second reception unit 50 receives the change operation with respect to the structure of the chemical substance displayed on the display 23.

Similarly to the first derivation unit 42, the second derivation unit 52 derives a second index value indicating the performance or the structure of the chemical substance after the change operation based on the structure data indicating the structure of the chemical substance after the change operation by the user.

In a case in which the first index value and the second index value are different from each other, the determination unit 54 determines whether or not the partial structure that contributes to a change in a value from the first index value to the second index value is present in the chemical substance after the change operation. Specifically, the determination unit 54 determines whether or not the partial structure included in the chemical substance after the change operation is present in the partial structure data 32 and whether or not the index value of the same type as the index value of which the value is changed is associated with the partial structure. Based on the determination, the determination unit 54 determines whether or not the partial structure that contributes to the change in the value from the first index value to the second index value is present. In a case of the determination, the determination unit 54 targets the partial structure influenced by the change operation. Examples of the partial structure influenced by the change operation include a partial structure including an added node, a partial structure including a substituted node, and a partial structure including a node bonded to the deleted node.

The second display control unit 56 performs control of displaying the structure of the chemical substance after the change operation and the second index value of the chemical substance after the change operation on the display 23. In addition, in a case in which the determination unit 54 determines that the partial structure that contributes to the change in the value from the first index value to the second index value is present in the chemical substance after the change operation, the second display control unit 56 performs control of highlighting the partial structure.

In addition, in a case in which the first index value and the second index value are different from each other, the second display control unit 56 performs control of displaying both the first index value and the second index value. In addition, the second display control unit 56 performs control of highlighting a portion in which the structure of the chemical substance is changed before and after the change operation.

Fig. 7 shows an example of a second display screen displayed on the display 23 by the control of the second display control unit 56. As shown in Fig. 7, the second display screen according to the present embodiment includes two display regions A1 and A2, similarly to the first display screen. A graph showing the structure of the chemical substance after the change operation is displayed in the display region A1. In addition, in the graph, the portion in which the structure of the chemical substance is changed before and after the change operation is highlighted. In the example of Fig. 7, the added node is highlighted by being filled with a color set in advance.

In addition, the second index value of the chemical substance after the change operation is displayed in the display region A2. In addition, in the display region A2, in a case in which the first index value and the second index value are different from each other, both the first index value and the second index value are displayed. Fig. 7 shows an example in which, in "index value 2", the first index value is "20" and the second index value is "30".

Also, in the display region A1, the partial structure that contributes to the change in the value from the first index value to the second index value is highlighted. Fig. 7 shows an example in which the partial structure surrounded by a broken line including the added node is the partial structure that contributes to the change in the value from the first index value to the second index value. For example, the partial structure is highlighted by filling the region surrounded by the broken line with a light transmittance set in advance and a color set in advance.

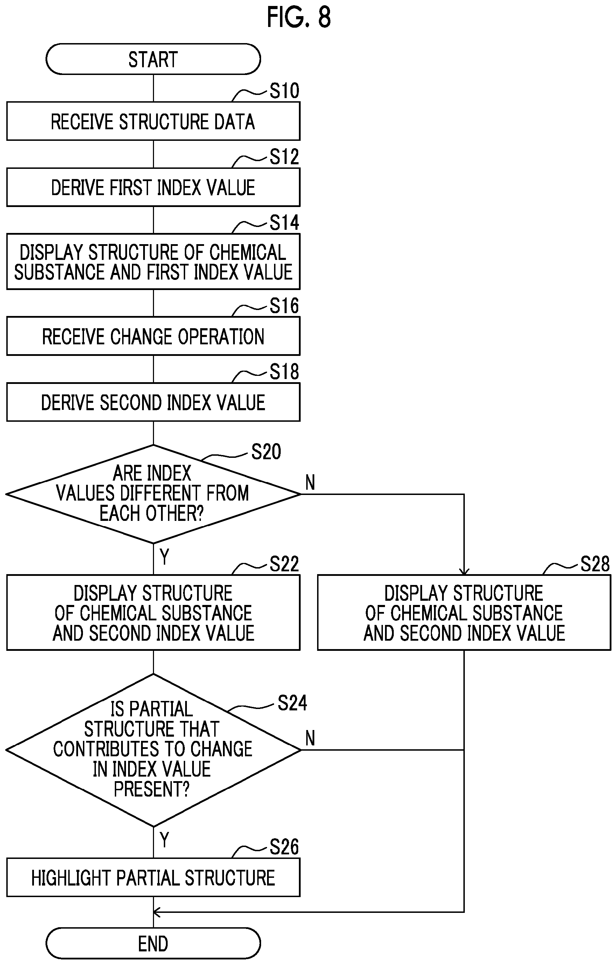

Next, with reference to Fig. 8, the operation of the information processing apparatus 10 according to the present embodiment will be described. The CPU 20 executes the information processing program 30 to execute highlighting processing shown in Fig. 8. The highlighting processing is executed, for example, in a case in which an execution instruction is input by the user via the input device 24.

In step S10 of Fig. 8, the first reception unit 40 receives the structure data indicating the structure of the chemical substance, which is the processing target and is designed by the user using the molecular design editor. In step S12, as described above, the first derivation unit 42 derives the first index value of the chemical substance, which is the processing target, based on the structure data received in step S10.

In step S14, the first display control unit 44 performs control of displaying the structure of the chemical substance, which is the processing target, indicated by the structure data received in step S10 and the first index value of the chemical substance derived in step S12 on the display 23. The first display screen shown in Fig. 6 as an example is displayed on the display 23 by the control.

In step S16, the second reception unit 50 receives the change operation with respect to the structure of the chemical substance displayed on the display 23 in step S14. In step S18, as described above, the second derivation unit 52 derives the second index value of the chemical substance after the change operation based on the structure data indicating the structure of the chemical substance after the change operation by the user.

In step S20, the determination unit 54 determines whether or not the first index value derived in step S12 and the second index value derived in step S18 are different from each other. In a case in which an affirmative determination is made in the determination, the processing proceeds to step S22. In step S22, the second display control unit 56 performs control of displaying the structure of the chemical substance after the change operation and the second index value of the chemical substance after the change operation on the display 23. In this case, the second display control unit 56 performs control of displaying both the first index value and the second index value, with respect to the index values in which the first index value and the second index value are different from each other. Further, in this case, the second display control unit 56 performs control of highlighting the portion in which the structure of the chemical substance is changed before and after the change operation.

In step S24, as described above, the determination unit 54 determines whether or not the partial structure that contributes to the change in the value from the first index value to the second index value is present in the chemical substance after the change operation, with reference to the partial structure data 32. In a case in which an affirmative determination is made in the determination, the processing proceeds to step S26. In step S26, the second display control unit 56 performs control of highlighting the partial structure that contributes to the change in the value from the first index value to the second index value. By the control of step S22 and step S26, the second display screen shown in Fig. 7 as an example is displayed on the display 23. In a case in which the processing of step S26 is terminated, the highlighting processing is terminated.

On the other hand, in a case in which a negative determination is made in the determination in step S20, the processing proceeds to step S28. In step S28, the second display control unit 56 performs control of displaying the structure of the chemical substance after the change operation and the second index value of the chemical substance after the change operation on the display 23. In this case, the second display control unit 56 performs control of highlighting the portion in which the structure of the chemical substance is changed before and after the change operation. In a case in which the processing of step S28 is terminated, the highlighting processing is terminated. In addition, in a case in which in a case in which a negative determination is made in the determination in step S24, the highlighting processing is terminated.

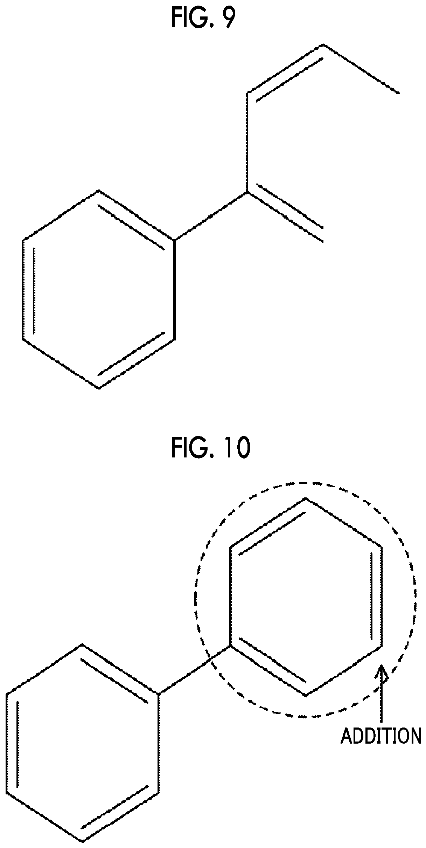

Next, with reference to Figs. 9 and 10, a specific example will be described. Here, an example will be described in which the user designs a benzene ring by using the molecular design editor. In addition, here, an example will be described in which the number of ring structures is used as the index value. In the partial structure data 32, it is assumed that the partial structure of "benzene ring" is associated with the type of the index value of "the number of ring structures" and the index value of " 1".

The user adds one carbon to the chemical substance shown in Fig. 9 by operating the molecular design editor. By the change operation with respect to the structure of the chemical substance, the structure of the chemical substance after the change is as shown in Fig. 10. By the change operation, the index value of "the number of ring structures" in the chemical substance is changed from "1" to "2". As described above, the partial structure data 32 includes a combination of the partial structure of "benzene ring", and the type of the index value of "the number of ring structures" and the index value "1".

That is, the determination unit 54 determines that the benzene ring containing the added carbon is the partial structure that contributes to the change in the value from the first index value to the second index value. Therefore, the benzene ring containing the added carbon is highlighted on the second display screen. In the example of Fig. 10, the benzene ring surrounded by a broken line circle is highlighted.

As described above, according to the present embodiment, it is possible to understand the influence of the chemical substance on the index value in a case in which the structure of the chemical substance is changed.

It should be noted that, in the embodiment described above, various processors shown below can be used as the hardware structure of processing units that execute various pieces of processing, such as the first reception unit 40, the first derivation unit 42, the first display control unit 44, the second reception unit 50, the second derivation unit 52, the determination unit 54, and the second display control unit 56. As described above, the various processors include, in addition to the CPU that is a general-purpose processor that executes software (program) to function as various processing units, a programmable logic device (PLD) that is a processor of which a circuit configuration can be changed after manufacture, such as a field programmable gate array (FPGA), and a dedicated electric circuit that is a processor having a circuit configuration that is designed for exclusive use in order to execute specific processing, such as an application specific integrated circuit (ASIC).

One processing unit may be configured by one of the various processors, or may be configured by a combination of two or more processors of the same type or different types (for example, a combination of a plurality of FPGAs, or a combination of the CPU and the FPGA). Further, a plurality of processing units may be configured by one processor.

A first example of the configuration in which the plurality of processing units are configured by one processor is a form in which one processor is configured by a combination of one or more CPUs and the software and this processor functions as the plurality of processing units, as represented by computers, such as a client and a server. A second example thereof is a form of using a processor that realizes the function of the entire system including the plurality of processing units by one integrated circuit (IC) chip, as represented by a system on chip (SoC) or the like. In this way, the various processing units are configured by using one or more of the various processors described above, as the hardware structure.

Further, more specifically, as the hardware structure of the various processors, an electric circuit (circuitry) in which circuit elements, such as semiconductor elements, are combined can be used.

In addition, in the embodiment described above, an aspect has been described in which the information processing program 30 is stored (installed) in the storage unit 22 in advance, but the present disclosure is not limited to this. The information processing program 30 may be provided in a form of being recorded in a recording medium, such as a compact disc read only memory (CD-ROM), a digital versatile disc read only memory (DVD-ROM), and a universal serial bus (USB) memory. Moreover, the information processing program 30 may be provided in a form being downloaded from an external device via a network.

The disclosure of JP2020-216937 filed on December 25, 2020 is incorporated in the present specification by reference in its entirety. Also, all documents, patent applications, and technical standards described in the present specification are incorporated in the present specification by reference to the same extent as a case in which each document, patent application, and technical standard are specifically and individually described to be incorporated by reference.

## Claims

1. An information processing apparatus comprising:
at least one processor,
wherein the processor
performs control of displaying a structure of a chemical substance, which is a processing target, and a first index value indicating performance or the structure of the chemical substance on a display device,
receives a change operation with respect to the structure of the chemical substance displayed on the display device, and
performs control of displaying a structure of the chemical substance after the change operation and a second index value indicating performance or the structure of the chemical substance after the change operation on the display device, and performs, in a case in which a partial structure that contributes to a change in a value from the first index value to the second index value is present in the chemical substance after the change operation, control of highlighting the partial structure.

2. The information processing apparatus according to claim 1,
wherein the processor performs control of displaying both the first index value and the second index value in a case in which the first index value and the second index value are different from each other.

3. The information processing apparatus according to claim 1 or 2,
wherein the processor determines that the partial structure that contributes to the change in the value from the first index value to the second index value is present, in a case in which the partial structure included in the chemical substance after the change operation is present in partial structure data in which a partial structure of the chemical substance and an index value indicating performance or a structure of the partial structure are associated with each other.

4. The information processing apparatus according to any one of claims 1 to 3,
wherein the processor further performs control of highlighting a portion in which the structure of the chemical substance is changed before and after the change operation.

5. An information processing method executed by a processor provided in an information processing apparatus, the method comprising:
performing control of displaying a structure of a chemical substance, which is a processing target, and a first index value indicating performance or the structure of the chemical substance on a display device;
receiving a change operation with respect to the structure of the chemical substance displayed on the display device; and
performing control of displaying a structure of the chemical substance after the change operation and a second index value indicating performance or the structure of the chemical substance after the change operation on the display device, and performing, in a case in which a partial structure that contributes to a change in a value from the first index value to the second index value is present in the chemical substance after the change operation, control of highlighting the partial structure.

6. An information processing program causing a processor provided in an information processing apparatus to execute a process comprising:
performing control of displaying a structure of a chemical substance, which is a processing target, and a first index value indicating performance or the structure of the chemical substance on a display device;
receiving a change operation with respect to the structure of the chemical substance displayed on the display device; and
performing control of displaying a structure of the chemical substance after the change operation and a second index value indicating performance or the structure of the chemical substance after the change operation on the display device, and performing, in a case in which a partial structure that contributes to a change in a value from the first index value to the second index value is present in the chemical substance after the change operation, control of highlighting the partial structure.
